**Europäisches Patentamt**

**European Patent Office**

**Office européen des brevets**

(19)

(11) **EP 0 823 215 A1**

(12) **EUROPEAN PATENT APPLICATION**

| | |
|---|---|
| (43) Date of publication:<br>**11.02.1998 Bulletin 1998/07** | (51) Int. Cl.⁶: **A01N 63/00**, C12N 1/20<br>// (C12N1/20, C12R1:01) |
| (21) Application number: **96112680.2** | |
| (22) Date of filing: **06.08.1996** | |

| | |
|---|---|
| (84) Designated Contracting States:<br>**IT**<br><br>(83) **Declaration under Rule 28(4) EPC (expert solution)**<br><br>(71) Applicant:<br>**BIO INTEGRATED TECHNOLOGY S.r.l.**<br>**06050 Todi (Perugia) (IT)** | (72) Inventors:<br>• **Ragni, Adriano**<br>  **06060 S. Feliciano (IT)**<br>• **Valentini, Federico**<br>  **06085 Perugia (IT)**<br>• **Fridlender, Bertold**<br>  **Jerusalem 96428 (IL)**<br><br>(74) Representative:<br>**VOSSIUS & PARTNER**<br>**Postfach 86 07 67**<br>**81634 München (DE)** |

(54) **Insecticidal bacteria**

(57) Provided are novel bacteria belonging to the species *Photorhabdus luminescens* which possess insecticidal activity which is not dependent on these bacteria being carried by a nematode infection vector. Live or inactivated *P.luminescens* cells, as well as fragments of such cells and supernatants of culture of insecticidal *P.luminescens* bacteria, are used in accordance with the invention as insecticidal agents for the treatment of insect infestation of plants.

**EP 0 823 215 A1**

## Description

The present invention is generally in the field of insecticides and concerns preparations and methods for combatting crop insect infestations. More specifically, the present invention provides such preparations and methods where the active ingredient is an insecticidal bacteria, dead bacterial cells or components thereof. The invention further concerns novel bacteria possessing an insecticidal activity.

Infestation of crops by insects is a major cause of reduction of crop yield. While chemical insecticides have been used for decades to combat such infestation, it is today a growing tendency, given the environmental hazards associated with the use of such chemicals, to shift towards biological control means.

Bacteria of the species *Photorhabdus* have the capability to infect insects with the infection having the effect of killing their insect host. *Photorhabdus* bacteria are carried by nematodes and their infectious life cycle involves the use of the nematodes as an infection vector. Indeed, it has been believed in the art that the *Photorhabdus* infection depends on the nematode host. Accordingly, it has been suggested to use *Photorhabdus* carrying nematodes as insecticidal agents (Bedding, R.A. and Miller, L.A. *Env. Entomol.*, **10**:449-453, 1981).

In accordance with the invention it has surprisingly been found that contrary to previous belief, certain *Photorhabdus luminescens* (*P.luminescens;* also known as *Xenorhabdus luminescens*) bacteria have the capacity to infect insects without dependency on a nematode carrier as an infection vector, In accordance with the invention some novel *P.luminescens* strains have been isolated and obtained in a pure form and found to be highly effective as insecticidal agents. Such bacteria, to be referred to herein at times as *"insecticidal bacteria",* may be used to combat insect infestation of crops in agriculture or horticulture.

In addition it was found in accordance with the invention that the insecticidal activity is also, at least partially, retained by inactivated bacterial cells, namely the insecticidal activity does not necessarily depend on the viability of the insecticidal bacteria. Still further, it was found that at least some insecticidal activity is also manifested by broken inactivated insecticidal bacteria, as well as by the supernatant obtained from a culture of the insecticidal bacteria.

The invention thus provides, by one of its aspects, an insecticidal composition, comprising insecticidally effective amounts of an active ingredient selected from the group consisting of:

i. bacteria having insecticidal activity belonging to the species *Photorhabdus luminescens*, the insecticidal activity being manifested without the need for a nematode as an infection vector;

ii. inactivated *P.luminescens* cells;

iii. fragments of inactivated *P.luminescens* cells; and

iv. supernatant of cultures of *P.luminescens* cells or a fraction thereof possessing the insecticidal activity.

A bacteria having insecticidal activity, in the context of the invention, is used to denote a bacteria which can infect an insect and cause its death, inhibition of its growth, effect its motility, etc. The end result of such insecticidal activity is a reduction for inhibition of insect infestation of the plants or crops. The insecticidal bacteria of the invention are unique in that the insecticidal activities are also manifested if applied by themselves without a nematode vector.

Also provided by the invention is a method for treatment of insect infestation of plants comprising administering to plants or to their surroundings an effective amount of said active ingredient.

The term *"treatment"* in the context of the invention should be understood as encompassing both treatment of an acute infestation as well as treatment intended for prevention of insect infestation prior to its occurrence.

The bacteria may be administered on either or both of the plants' aerial parts as well as to the ground. The administration may be by spraying, by delivering the composition through the irrigation water, as well as by administration of a composition in a dry particulate or powder form.

The composition may either contain said active ingredient alone or in combination with one or more additional insecticidal agents such as another insecticidal microorganism or chemical insecticide, in both cases together with a suitable carrier. Such a carrier may be any one of those known in the art, e.g. natural or regenerated mineral substances, diluents, dispersants, wetting agents, tackifires, binders or fertilizers.

The present invention also provides by a further of its aspects, a pure culture of novel insecticidal bacteria of the species *P.luminescens*. Particularly preferred are such bacteria belonging to a new strain purified in accordance with the invention which is termed herein as *"XP01"*. The XP01 strain has been deposited in the Collection Nationale de Cultures de Microorganismes (CNCM), Institut Pasteur, 25, rue du Dr. Roux, Paris 15ème having the CNCM Accession No. I-1760 with the deposit date being July 23, 1996. It will be appreciated that this strain is but an example and other strains of *P.luminescens* which possess an insecticidal activity may also be used in accordance with the invention.

For treatment, an effective amount of said active ingredient will be applied onto plants, plant parts, or introduced into the plant environment, particularly into the soil. An effective amount is to be understood as an amount of said active ingredient sufficient to cause mortality of a substantial portion of the insects, an amount effective in reducing the level of activity of the insects, an amount effective in reducing the average weight of the insect, an amount effective in reducing the insect biomass, etc. Where said active ingredient is used for prevention of insect infestation, an effective amount would be an amount which is effective in inhibiting development of the insects to yield a population which will be damaging to the plants or the plants' crops.

Said active ingredient may be applied in either acute or preventive treatment, in a number of administration modes. For example, said active ingredient may be mixed with the irrigation water and thus be applied to either aerial parts of plants or to the soil (depending on the type of irrigation) via the irrigation water. By another example, liquid formulation comprising said active ingredient may be sprayed, particularly onto aerial plant parts, e.g. using conventional sprayers. By another example, said active ingredient may be mixed with soil, e.g. pot soil, for the purpose of prevention of insecticidal infestation of the plants after planting. By a further example, plant parts, and particularly seeds, are impregnated with a solution comprising said active ingredient. The active ingredient may also be used for post-harvest protection of crops, which may be achieved by spraying or impregnating the crops with the active ingredient, either pre or after harvest.

As will be appreciated by the artisan, the treatment regime of said active ingredient will vary depending oil the kind of insects to be controlled, the nature of the plants to be treated, the method of application of the composition comprising the bacteria, etc. In some cases, a single treatment may suffice, while in other cases, the infected plants will have to be treated over a period of time by recurrent application of the composition. The dose of the administered active ingredient within a treatment will also vary depending on the above and other factors.

The bacteria of the invention may be obtained by isolation from cadavers of larvae of insects which were incubated with nematodes carrying insecticidal bacteria. Haemolymph obtained from the larvae cadavers may be incubated in a suitable medium wherein bacterial colonies develop and single bacterial colonies may then be selected and purified.

The insecticidal bacteria may be cultivated to obtain large amounts by fermentation in a suitable growth medium for various periods of time. The fermented broth which is harvested after fermentation and contains large amounts of the selected bacteria will hereinafter be referred to as *"ferment"*.

Said active agent was found to be effective in particular against Lepidoptera such as *Mamestra brassicae, Spodoptera littoralis, Helicoverpa armigera*, *Agrotis ipsilon*, *Scotia segetum* and *Lobesia botrana*.

The invention will be illustrated further by some specific embodiments described in the following examples and in the annexed drawings:

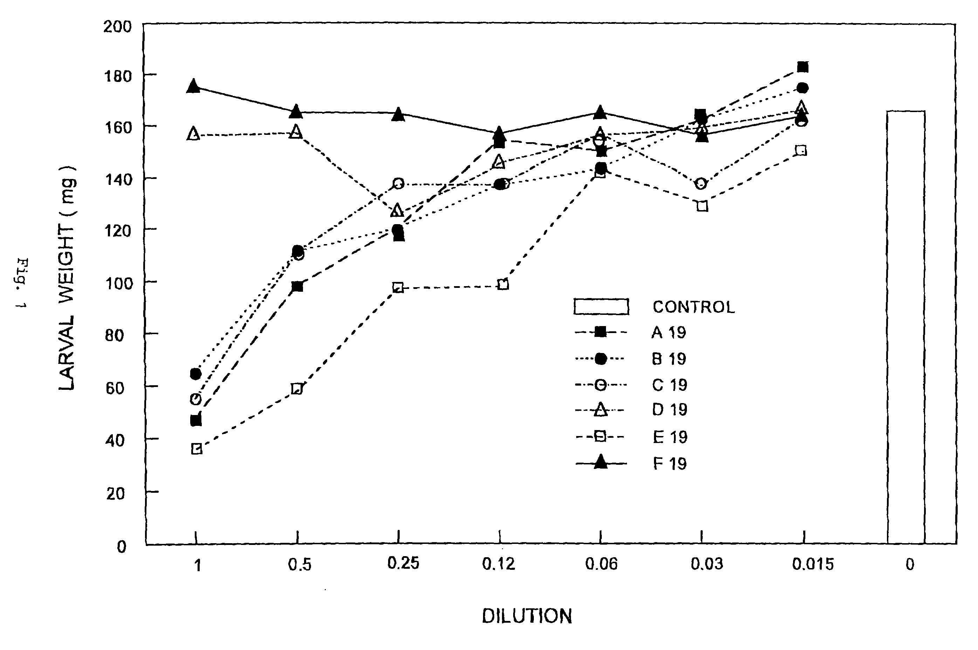

The Figure shown in the annexed drawing is a graphic representation of results of an experiment in which the insecticidal activity of different preparations derived from various dilutions of *P.luminescens* XP01 bacteria was tested. The effect which was tested was a change in weight of 5-days old *Mamestra brassicae* larvae five days after administration of the preparation (the initial weight of the larvae averaged 6.33 mg). The *P.luminescens* ferment from which the preparation were derived was a 19 hour culture. Different samples were tested as follows:

**Sample A19**:  Cells from an XP01 ferment washed three times in a basic Ringer solution (pH 9);
**Sample B19**:  Cells from an XP01 ferment washed three times in an acid Ringer solution (pH 5);
**Sample C19**:  Cells from an XP01 ferment washed three times in a neutral Ringer solution (pH 7);
**Sample D19**:  Supernatant of an XP01 ferment filtered on a 22 micron filter (to remove bacterial cells from the suspension);
**Sample E19**:  Non treated whole XP01 culture.
**Sample F19**:  R5 medium not containing XP01 cells incubated for 19 hours at 25°C.

The following examples illustrate the invention.

In some examples below, the insecticidal activity against the various insects was determined by measuring the mortality score (%) of the neonate larvae incubated with the tested preparation. The scored mortality of the treated larvae was corrected to take into consideration the mortality of untreated control larvae using Abbott's formula as follows:

$$\% \text{ Corrected mortality} = \frac{\% \text{ test mortality - } \% \text{ control mortality}}{100 - \% \text{ control mortality}} \times 100$$

**Example 1**

The XP01 strain of *Photorhabdus luminescens* XP01 was isolated from cadavers of *Galleria mellonella* as follows:

i. 10 last instar larvae of *G. mellonella* were placed into a petri dish with moist filter paper and approximately 1500 Dauer Juveniles of *Heterorhabditis bacteriophora* strain were added. The petri dish containing the larvae and the nematodes was then incubated at 25°C in the dark.
ii. At the moment of insect death (after approx. 2 days of incubation), the cadavers were washed in a petri dish with 70% methanol for 5 mins. and then rinsed in sterile demineralized water.

iii. Each insect cadaver was opened with the help of a scissor and a needle. A drop of haemolymph from each cadaver was streaked with a sterile loop on a nutrient agar plate.

iv. The nutrient agar plates were incubated at 25°C in the dark for 48 hours. After morphological observation of the developed colonies and microscopical observation of the bacteria the single *P.luminescens* colonies were selected and streaked on NBTA, McConkey and nutrient agar plates.

v. The sub-culture of colonies as described in Step No. iv above was repeated several times in order to avoid contaminants and to ensure selection of a single colony of *Photorhabdu*s.

A pure culture of a novel strain of *P.luminescens* was obtained. One of such culture, termed *"XP01"*, was deposited at the Collection Nationale de Cultures de Microorganismes (CNCM) on July 23, 1996, under Accession No. I-1760.

**Example 2**

Serial dilutions of XP01 ferments were prepared using demineralized water as the diluent and the whole culture and each of the dilutions were incubated with *Mamestra brassicae* neonates. Mortality of the larvae was scored after 4 days of incubation at 27°C. The results are shown in the following Table 1:

Table 1

| Insecticidal efficacy of the raw bacterial suspension against neonate of *Mamestra brassicae* at several dilution rates | | | | |
| --- | --- | --- | --- | --- |
| Treatment | Dilution | # insects | # dead | % mortality (corrected by Abbott's formula except control) |
| Control (untreated) | - | 64 | 2 | 3.1 |
| XP01 ferment | non-diluted | 31 | 31 | 100 |
| XP01 ferment | 1:10 | 32 | 30 | 93.6 |
| XP01 ferment | 1:100 | 32 | 8 | 22.6 |
| XP01 ferment | 1:1000 | 32 | 1 | 0 |

As seen in the above Table 1, the XP01 ferment showed insecticidal activity against the neonate larvae of *M. brassicae* as measured by the percent mortality of the neonate larvae.

**Example 3**

Samples of ferments of XP01 fermented in R5 medium at 25°C, were taken at 19, 24 and 40 hours from the beginning of the fermentation. Part of the sample taken at 19 hours was centrifuged on a bench centrifuge at 15000 rpm for 5 mins. and then the supernatant was separated from the bacterial pellet.

The pellets from the 19 hours ferment were divided into six samples:

**Sample A19**:   Was washed three times in a basic Ringer solution (pH 9);
**Sample B19**:   Was washed three times in an acid Ringer solution (pH 5);
**Sample C19**:   Was washed three times in a neutral Ringer solution (pH 7);
**Sample D19**:   The supernatant was filtered through an 0.22 micron filter to remove bacterial cells from the suspension;
**Sample E19**:   Original ferment (whole bacterial culture);
**Sample F19**:   R5 medium which did not contain XP01 culture incubated for 19 hours at 25°C.

The samples were bioassayed against 5 day old *Mamestra brassicae* larvae similarly as in Example 2, and incubated for 6 days after which mortality was scored and each dose group of the surviving larvae was weighed and the average weight of the larvae calculated. As seen in the Figure, a reduction in the weight of the larvae treated with the Samples A19, B19, C19 and E19 was detected which was inversely proportional with the dilution of the samples. No reduction was observed using Samples D19 and F19.

The 19 hours ferment (Sample E19), the 24 hours ferment (Sample E24) and the 40 hours ferment (Sample E40) were bioassayed against neonate *Mamestra brassicae* similarly as above, the mortality was scored after 6 days and Lethal Dilution Rate 50% (LDR50) was calculated by probit analysis or estimated by graphical interpolation. As seen in Tables 2-4 below, the insecticidal activity of the XP01 whole culture was not affected by the fermentation length of the culture and there were no significant differences between the cultures fermented for 19, 24 or 40 hours.

Table 2

| (Neonates - 19 hours) Insecticidal efficacy of the raw bacterial suspension (harvested after 19 hours of incubation) against neonate of *Mamestra brassicae* at several dilution rates | | | | |
|---|---|---|---|---|
| Treatment | Dilution | # insects | % dead | % mortality (corrected by Abbott's formula except control) |
| Control (untreated) | - | 64 | 7 | 10.9 |
| E19 | non-diluted | 32 | 32 | 100 |
| E19 | 1:2 | 32 | 31 | 96.5 |
| E19 | 1:4 | 32 | 32 | 100 |
| E19 | 1:8 | 32 | 32 | 100 |
| E19 | 1:16 | 31 | 15 | 42.1 |
| E19 | 1:32 | 32 | 9 | 19.3 |
| E19 | 1:64 | 32 | 9 | 19.3 |
| E19 | 1:128 | 31 | 5 | 5.9 |

Table 3

| (Neonates - 24 hours) Insecticidal efficacy of the raw bacterial suspension (harvested after 24 hours of Incubation) against neonate of *Mamestra brassicae* at several dilution rates | | | | |
|---|---|---|---|---|
| Treatment | Dilution | # insects | # dead | % mortality (corrected by Abbott's formula except control) |
| Control (untreated) | - | 64 | 12 | 18.8 |
| E24 | non-diluted | 32 | 32 | 100 |
| E24 | 1:2 | 32 | 30 | 92.3 |
| E24 | 1:4 | 32 | 30 | 92.3 |
| E24 | 1:8 | 32 | 28 | 84.6 |
| E24 | 1:16 | 32 | 25 | 73.1 |
| E24 | 1:32 | 32 | 10 | 15.3 |
| E24 | 1:64 | 32 | 6 | 0 |
| E24 | 1:128 | 32 | 4 | 0 |

Table 4

| C - (Neonates - 40 hours) Insecticidal efficacy of the raw bacterial suspension (harvested after 40 hours of incubation) against neonate of *Mamestra brassicae* at several dilution rates | | | | |
|---|---|---|---|---|
| Treatment | Dilution | # insects | # dead | % mortality (corrected by Abbott's formula except control) |
| Control (untreated) | - | 64 | 2 | 3.1 |
| E40 | non-diluted | 32 | 32 | 100 |
| E40 | 1:2 | 32 | 32 | 100 |
| E40 | 1:4 | 32 | 29 | 90.3 |
| E40 | 1:8 | 32 | 30 | 93.6 |
| E40 | 1:16 | 32 | 19 | 58.1 |
| E40 | 1:32 | 32 | 8 | 22.6 |
| E40 | 1:64 | 32 | 2 | 3.2 |
| E40 | 1:128 | 32 | 1 | 0 |

The approximate LDR50 for the different preparations was as follows:

E19 -    about 1:17.2 dilutions
E24 -    about 1:18.5 dilutions
E40 -    about 1:18.2 dilutions

**Example 4**

The insecticidal activity of XP01 ferments was tested against neonate larvae of four different Lepidopterian species: (*S. littoralis, H. armigera, A. ipsilon* and *S. segetum*).

Several samples, each containing 6 ml of 24 hour XP01 ferments, (the fermentation being for 24 hours at 25°C in an R5 medium) were prepared. Diluted and non diluted samples were incubated with neonates of the four Lepidopterian species.

As seen in Tables 5-8 below, XP01 ferments showed an insecticidal activity against all four Lepidopterian species.

Table 5

| (*Spodoptera littoralis* neonates) Insecticidal efficacy of the raw bacterial suspension against neonate of *Spodoptera littoralis* at four dilution rates | | | | |
|---|---|---|---|---|
| Treatment | Dilution | # insects | # dead | % mortality (corrected by Abbott's formula except control) |
| Control (untreated) | - | 64 | 2 | 3.1 |
| E24 | non-diluted | 32 | 22 | 67.8 |
| E24 | 1:4 | 32 | 15 | 45.2 |
| E24 | 1:16 | 32 | 2 | 3.2 |
| E24 | 1:64 | 32 | 1 | 0 |

Table 6

| (*Agrotis ipsilon* neonates) Insecticidal efficacy of the raw bacterial suspension against neonate of *Agrotis ipsilon* at four dilution rates | | | | |
|---|---|---|---|---|
| treatment | dilution | # insects | # dead | % mortality (corrected by Abbott's formula except control) |
| Control (untreated) | - | 64 | 1 | 1.6 |
| E24 | non-diluted | 32 | 30 | 93.6 |
| E24 | 1:4 | 32 | 18 | 55.5 |
| E24 | 1:16 | 32 | 8 | 23.8 |
| E24 | 1:64 | 32 | 4 | 11.1 |

Table 7

| (*Helicoverpa armigera* neonates) Insecticidal efficacy of the raw bacterial suspension against neonate of *Helicoverpa armigera* at four dilution rates | | | | |
|---|---|---|---|---|
| Treatment | Dilution | # insects | # dead | % mortality (corrected by Abbott's formula except control) |
| Control (untreated) | - | 64 | 1 | 1.6 |
| E24 | non-diluted | 32 | 31 | 96.8 |
| E24 | 1:4 | 32 | 26 | 11.1 |
| E24 | 1.16 | 32 | 4 | 0 |
| E24 | 1:64 | 32 | 0 | 0 |

Table 8

| (*Scotia segetum* neonates) Insecticidal efficacy of the raw bacterial suspension against neonate of *Scotia segetum* at four dilution rates | | | | |
|---|---|---|---|---|
| Treatment | Dilution | # insects | # dead | % mortality (corrected by Abbott's formula) |
| Control (untreated) | - | 64 | 0 | 0 |
| E24 | non-diluted | 32 | 32 | 100 |
| E24 | 1:4 | 32 | 31 | 96.9 |
| E24 | 1:16 | 32 | 8 | 25 |
| E24 | 1:64 | 32 | 3 | 9.4 |

**Example 5**

Ferments of XP01, fermented for 24 hours at 25°C in R5 medium were obtained and the following samples were prepared:

**Sample A**: Non treated ferments;
**Sample B**: R5 medium containing no bacteria but incubated for 24 hours at 25°C;
**Sample C**: Supernatant of the ferments filtrated through a 22 micron filter;
**Sample D**: Bacterial pellets of the ferments washed three times in isotonic solution and then resuspended in isotonic solution;
**Sample E**: Bacterial pellets as in D resuspended in a medium having a pH 4.5;
**Sample F**: Demineralized water;
**Sample G**: Bacterial pellets of the ferments crushed in liquid nitrogen in a mortar with pestle;
**Sample H**: Supernatant of a resuspended pellet of the whole bacterial culture which was crushed in liquid nitrogen in a mortar with pestle (Sample G) filtered on a 22 micron filter.

All the above samples were incubated with neonate larvae of *M. brassicae* at 27°C for a period of 5 days. The results are shown in the following Table 9 and the Probit analysis in Table 10 below:

As seen in Table 9 below, the highest insecticidal activity detected was that of the whole XP01 culture. The supernatant of the whole bacterial culture which was filtrated through a 22 micron filter (Sample C) showed no insecticidal activity against these neonate larvae. Against this, the supernatant produced from the bacterial cell pellet of the cells which were lightly damaged by liquid nitrogen treatment (Sample H) showed an insecticidal activity. This supports the possibility that the bacterial cells comprise an insecticidal component which upon cell damage leaks into the supernatant.

## Table 9

Insecticidal efficacy of the raw bacterial suspension and some derivatives
derivatives against neonate of Mamestra brassicae at several dilution rates

| Treatment | Dilution | # insects | # dead | % mortality (corrected by Abbott's formula except control) |
|---|---|---|---|---|
| Control (untreated) | – | 64 | 5 | 7.8 |
| A | non-diluted | 32 | 32 | 100 |
| A | 1:2 | 32 | 31 | 96.6 |
| A | 1:4 | 32 | 30 | 93.2 |
| A | 1:8 | 32 | 29 | 89.8 |
| A | 1:16 | 32 | 15 | 42.4 |
| A | 1:32 | 32 | 6 | 11.9 |
| A | 1:64 | 32 | 0 | 0 |
| A | 1:128 | 32 | 0 | 0 |
| B | non-diluted | 32 | 0 | 0 |
| B | 1:2 | 32 | 0 | 0 |
| B | 1:4 | 32 | 2 | 0 |
| B | 1:8 | 32 | 1 | 0 |
| B | 1:16 | 32 | 0 | 0 |
| B | 1:32 | 32 | 0 | 0 |
| B | 1:64 | 32 | 0 | 0 |
| B | 1:128 | 32 | 1 | 0 |
| C | non-diluted | 32 | 2 | 0 |
| C | 1:2 | 32 | 1 | 0 |
| C | 1:4 | 32 | 0 | 0 |
| C | 1:8 | 32 | 1 | 0 |
| C | 1:16 | 32 | 0 | 0 |
| C | 1:32 | 32 | 0 | 0 |
| C | 1:64 | 32 | 1 | 0 |
| C | 1:128 | 32 | 0 | 0 |

| Treatment | Dilution | # insects | # dead | % mortality (corrected by Abbott's formula except control) |
|-----------|----------|-----------|--------|-----------|
| D | non-diluted | 32 | 31 | 96.6 |
| D | 1:2 | 32 | 29 | 89.8 |
| D | 1:4 | 32 | 22 | 66.1 |
| D | 1:8 | 32 | 24 | 72.9 |
| D | 1:16 | 32 | 11 | 28.8 |
| D | 1:32 | 32 | 4 | 5.1 |
| D | 1:64 | 32 | 0 | 0 |
| D | 1:128 | 32 | 0 | 0 |
| E | non-diluted | 32 | 32 | 100 |
| E | 1:2 | 31 | 26 | 79.7 |
| E | 1:4 | 31 | 22 | 66.1 |
| E | 1:8 | 32 | 19 | 55.9 |
| E | 1:16 | 32 | 8 | 18.7 |
| E | 1:32 | 32 | 5 | 8.5 |
| E | 1:64 | 32 | 1 | 0 |
| E | 1:128 | 32 | 2 | 0 |
| F | non-diluted | 32 | 5 | 8.5 |
| F | 1:2 | 32 | 1 | 0 |
| F | 1:4 | 32 | 1 | 0 |
| F | 1:8 | 32 | 1 | 0 |
| F | 1:16 | 32 | 1 | 0 |
| F | 1:32 | 32 | 1 | 0 |
| F | 1:64 | 32 | 0 | 0 |
| F | 1:128 | 32 | 2 | 0 |

| Treatment | Dilution | # insects | # dead | % mortality (corrected by Abbott's formula except control) |
|---|---|---|---|---|
| G | non–diluted | 32 | 28 | 86.4 |
| G | 1:2 | 32 | 26 | 79.7 |
| G | 1:4 | 32 | 25 | 76.3 |
| G | 1:8 | 32 | 12 | 32.2 |
| G | 1:16 | 32 | 7 | 15.3 |
| G | 1:32 | 32 | 2 | 0 |
| G | 1:64 | 32 | 1 | 0 |
| G | 1:128 | 32 | 2 | 0 |
| H | non–diluted | 32 | 18 | 52.5 |
| H | 1:2 | 32 | 11 | 28.8 |
| H | 1:4 | 32 | 2 | 0 |
| H | 1:8 | 32 | 1 | 0 |
| H | 1:16 | 32 | 3 | 0 |

Table 10

| Probit analysis of results of Table 9 | | |
|---|---|---|
| A | subjects 256 | slope = 3.044±.353 |
| | LDR50=0.068 | limits: .043 to .104 |
| D | subjects 256 | slope = 1.169±.233 |
| | LDR50=.113 | limits: 0.68 to .183 |
| E | subjects 254 | slope = 2.200±.287 |
| | LDR50=.136 | limits: .101 to .177 |
| G | subjects 256 | slope = 2.033±.257 |
| | LDR50=.185 | limits: .122 to .272 |
| Estimated LDR50 of treatment H= .89 limits: .7 to 1.5 | | |

## Example 6

The insecticidal activity of a whole bacterial culture of XP01 bacteria was tested against neonates of *H. armigera*. XP01 culture was fermented for 24 hours at 25°C in R5 medium and from the ferments the following samples were prepared:

**Sample 1**: Bacterial pellet was prepared and exposed to osmotic shock by resuspension in 60 ml of demineralized water for 2 hours.

**Sample 2**: A pellet of the XP01 ferment was dried at 50°C for 9 hours, resuspended in an isotonic solution and sonicated to disrupt the cell walls;

**Sample 3**: A pellet of the ferment was treated as described for Sample 3 with a difference that the resuspension was in an acidic solution at pH 4.5.

**Sample 4**: The original ferment was tested against neonate larvae of *H. armigera* in an eight-dose bioassay.

The results from Samples 1-3 are shown in the following Table 11 and those of Sample 4 in Table 13 below. The Probit analysis is shown in Tables 12 and 14, respectively.

Table 11

| Insecticidal efficacy of processes samples from the raw bacterial suspension against neonate of *Mamestra brassicae* at eight dilution rates | | | | |
|---|---|---|---|---|
| **Treatment** | **Dilution** | **# insects** | **# dead** | **% mortality (corrected by Abbott's)** |
| Control (untreated) | - | 64 | 0 | 0 |
| Sample 1 | non-diluted | 32 | 29 | 90.6 |
| Sample 1 | 1:2 | 32 | 25 | 78.1 |
| Sample 1 | 1:4 | 32 | 10 | 31.2 |
| Sample 1 | 1:8 | 32 | 5 | 15.6 |
| Sample 1 | 1:16 | 32 | 0 | 0 |
| Sample 1 | 1:32 | 31 | 3 | 9.7 |
| Sample 1 | 1:64 | 32 | 2 | 6.2 |
| Sample 1 | 1:128 | 32 | 0 | 0 |
| Sample 2 | non-diluted | 32 | 20 | 62.5 |
| Sample 2 | 1:2 | 32 | 24 | 75 |
| Sample 2 | 1:4 | 32 | 15 | 46.9 |
| Sample 2 | 1:8 | 32 | 9 | 28.1 |
| Sample 2 | 1:16 | 32 | 3 | 9.4 |
| Sample 2 | 1:32 | 31 | 0 | 0 |
| Sample 2 | 1:64 | 32 | 0 | 0 |
| Sample 2 | 1:128 | 32 | 0 | 0 |
| Sample 3 | non-diluted | 32 | 24 | 75 |
| Sample 3 | 1:2 | 32 | 21 | 65.6 |
| Sample 3 | 1:4 | 32 | 16 | 50 |
| Sample 3 | 1:8 | 32 | 9 | 28.1 |
| Sample 3 | 1:16 | 32 | 2 | 6.2 |
| Sample 3 | 1:32 | 31 | 0 | 0 |
| Sample 3 | 1:64 | 32 | 0 | 0 |
| Sample 3 | 1:128 | 32 | 1 | 3.1 |

Table 12

| Probit analysis of results of Table 11 | | |
|---|---|---|
| S1 | subjects 256 | slope = 1.875±.211 |
| | **LDR50=.291** | limits: .149 to .799 |
| S2 | subjects 256 | slope = 1.718±.212 |
| | **LDR50=.343** | limits: .219 to .622 |
| S3 | subjects 256 | slope = 1.578±183 |
| | **LDR50=.260** | limits: .131 to .692 |

Table 13

| Insecticidal efficacy of the raw bacterial suspension against neonate of *Helicoverpa armigera* at eight dilution rates | | | | |
|---|---|---|---|---|
| **Treatment** | **Dilution** | **# insects** | **# dead** | **% mortality (corrected by Abbott's formula except control)** |
| Control (untreated) | - | 64 | 0 | **0** |
| Sample 4 | non-diluted | 32 | 28 | **87.5** |
| Sample 4 | 1:2 | 32 | 26 | **81.2** |
| Sample 4 | 1:4 | 32 | 26 | **81.2** |
| Sample 4 | 1:8 | 32 | 7 | **21.9** |
| Sample 4 | 1:16 | 32 | 14 | **43.8** |
| Sample 4 | 1:32 | 31 | 7 | **21.9** |
| Sample 4 | 1:64 | 32 | 0 | **0.0** |
| Sample 4 | 1:128 | 32 | 2 | **6.2** |

Table 14

| Probit analysis of results of Table 13 | | |
|---|---|---|
| Sample 4 | subjects 256 | slope = 1.483±.160 |
| | **LDR50=0.135** | limits: 0.068 to 0.293 |

As seen in Table 11, the XP01 cell culture shows an insecticidal activity against larvae of *M. brassicae* in correlation with the dilution of the tested composition (Samples 1, 2, 3).

As seen in Table 13, an XP01 whole culture used in an 8-dose bioassay showed insecticidal activity against neonates of *Helicoverpa armigera* (Sample 4) although to a different extent than the insecticidal activity of the same bacteria against neonates of *M. brassicae*.

**Example 7**

The insecticidal activity of XP01 cells inactivated by formaline against neonates of *M. brassicae* was tested.

XP01 was fermented in an R5 medium at 25°C, samples of the ferment were taken and treated as follows:

**Sample A**:   Untreated ferment.
**Sample B**:   Ferment treated with 3μl/ml of 30% (v/v) formaline.
**Sample C**:   Ferment treated with 6μl/ml of 30% (v/v) formaline.

The activity of these samples was tested versus the following control preparations:

**Control A**:   Deionized water.
**Control B**:   Deionized water with 3μl/ml of 30% (v/v) formaline.
**Control C**:   Deionized water with 6μl/ml of 30% (v/v) formaline.

In order to control the efficacy of formaline to kill the bacteria, cells from Sample A, B and C, were streaked on 3 nutrient agar plates that were incubated at 25°C in the dark for 2 days. This test confirmed that all bacterial cells were killed by the formaline.

The above controls and samples were tested in a bioassay against neonates of *M. biassicae*.

The results summarized in Table 15 show that XP01 cells inactivated by formaline maintain their insecticidal activity against neonates of *M. brassicae*.

Table 15

| Treatment | Dilution | # insects | # dead | % mortality (corrected by Abbott's formula) |
|-----------|----------|-----------|--------|---------------------------------------------|
| Control A | - | 32 | 0 | 0 |
| Control B | - | 32 | 0 | 0 |
| Control C | - | 32 | 0 | 0 |
| Sample A | 1:1 | 32 | 24 | 75.0 |
| Sample A | 1:2 | 32 | 27 | 84.4 |
| Sample A | 1:4 | 31 | 17 | 54.8 |
| Sample A | 1:8 | 32 | 15 | 46.9 |
| Sample A | 1:16 | 32 | 1 | 3.1 |
| Sample A | 1:32 | 31 | 2 | 6.2 |
| Sample A | 1:64 | 32 | 3 | 9.4 |
| Sample A | 1:128 | 32 | 1 | 3.1 |
| Sample B | 1:1 | 32 | 28 | 87.5 |
| Sample B | 1:2 | 32 | 27 | 84.4 |
| Sample B | 1:4 | 32 | 28 | 87.5 |
| Sample B | 1:8 | 32 | 20 | 62.5 |
| Sample B | 1:16 | 32 | 3 | 9.4 |
| Sample B | 1:32 | 32 | 1 | 3.1 |
| Sample B | 1:64 | 32 | 0 | 0 |
| Sample B | 1:128 | 32 | 0 | 0 |
| Sample C | 1:1 | 32 | 30 | 93.8 |
| Sample C | 1:2 | 32 | 29 | 90.6 |
| Sample C | 1:4 | 32 | 21 | 65.6 |
| Sample C | 1:8 | 32 | 14 | 43.7 |
| Sample C | 1:16 | 30 | 3 | 10.0 |
| Sample C | 1:32 | 31 | 1 | 3.2 |
| Sample C | 1:64 | 32 | 0 | 0 |
| Sample C | 1:128 | 32 | 0 | 0 |

**Example 8**

The insecticidal activity of XP01 fermentate against neonates of *L. botrana* was tested as follows:

XP01 culture was fermented in R5 medium at 25°C for 24 hours, after which a sample of the ferment was taken and used in one-dose bioassays against *L. botrana* neonates.

As seen in Table 16 below, the whole culture of XP01 showed insecticidal activity against neonates of *L. botrana*.

Table 16

| Treatment | Dilution | # insects | # insects dead | % mortality (corrected by Abbott's formula except control) |
|---|---|---|---|---|
| Control | - | 31 | 2 | 6.5 |
| XP01 | 1:1 | 32 | 24 | 73.3 |

**Example 9**

The insecticidal activity of whole culture of XP05 and XP98 against neonate larvae of *M. brassicae*, *H. armigera*, *A. ipsilon*, *S. segetum*, *S. littoralis L. botrana* was tested and compared to the insecticidal activity of whole culture of XP01 against larvae of the same kind.

A bacteria strain termed *"XP05"*, was isolated, as described in Example 1 for strain XP01, from haemolymph of *Galleria mellonella* larvae infected by Dauer Juveniles of *Steinernema feltiae* strain, UK. The bacteria XP98 was isolated in a similar manner also from haemolymph of *Galleria mellonella* larvae infected by Dauer Juveniles of *Steinernema* sp. strain 98.

XP01, XP05, XP98 were fermented in R5 media at 25°C in a 24 hour fermentation run.

The ferments were harvested and used without any dilution in a one-dose bioassays against neonates of *M. brassicae, H. armigera, A. ipsilon, S. segetum, S. littoralis L. botrana*. The results are shown in the following Tables 17-22.

Table 17

| One-dose bioassays against neonates of *M. brassicae* | | | | |
|---|---|---|---|---|
| Treatment | Dilution | # insects | # insects dead | % mortality (corrected by Abbott's formula) |
| Control | - | 32 | 0 | 0 |
| XP05 | 1:1 | 32 | 1 | 3.1 |
| XP98 | 1.1 | 32 | 3 | 9.4 |
| XP01 | 1:1 | 32 | 24 | 75.0 |

Table 18

| One-dose bioassays against neonates of *H. armigera* | | | | |
|---|---|---|---|---|
| Treatment | Dilution | # insects | # insects dead | % mortality (corrected by Abbott's formula) |
| Control | - | 32 | 0 | 0 |
| XP05 | 1:1 | 32 | 0 | 0 |
| XP98 | 1:1 | 32 | 0 | 0 |
| XP01 | 1:1 | 32 | 24 | 75.0 |

Table 19

| One-dose bioassays against neonates of *L. botrana* | | | | |
|---|---|---|---|---|
| Treatment | Dilution | # insects | # insects dead | % mortality (corrected by Abbott's formula except control) |
| Control | - | 31 | 2 | 6.5 |
| XP05 | 1:1 | 32 | 3 | 3.1 |
| XP98 | 1:1 | 32 | 1 | 0 |
| XP01 | 1:1 | 32 | 24 | 73.3 |

Table 20

| One-dose bioassays against neonates of *A. ipsilon* | | | | |
|---|---|---|---|---|
| Treatment | Dilution | # insects | # insects dead | % mortality (corrected by Abbott's formula except control) |
| Control | - | 32 | 1 | 3.1 |
| XP05 | 1:1 | 32 | 4 | 9.7 |
| XP98 | 1:1 | 32 | 1 | 0 |
| XP01 | 1:1 | 32 | 30 | 93.6 |

Table 21

| One-dose bioassays against neonates of *S. segetum* | | | | |
|---|---|---|---|---|
| Treatment | Dilution | # insects | # insects dead | % mortality (corrected by Abbott's formula) |
| Control | - | 32 | 0 | 0 |
| XP05 | 1:1 | 31 | 5 | 16.1 |
| XP98 | 1:1 | 32 | 3 | 9.4 |
| XP01 | 1:1 | 32 | 31 | 96.9 |

Table 22

| One-dose bioassays against neonates of *S. littoralis* | | | | |
|---|---|---|---|---|
| Treatment | Dilution | # insects | # insects dead | % mortality (corrected by Abbott's formula except control) |
| Control | - | 31 | 1 | 3.2 |
| XP05 | 1:1 | 32 | 2 | 3.1 |
| XP98 | 1:1 | 32 | 4 | 9.4 |
| XP01 | 1.1 | 32 | 24 | 74.2 |

As seen in Tables 17-22, while the insecticidal activity of XP01 against the 6 Lepidoterian species was apparent, the XP05 and XP98 strains have a small activity against the 6 Lepidoterian species.

**Claims**

1. An insecticidal composition, comprising insecticidally effective amounts of an active ingredient selected from the group consisting of:

    i. bacteria having insecticidal activity belonging to the species *P.luminescens*, the insecticidal activity being manifested without the need for a nematode as an infection vector;
    ii. inactivated *P.luminescens* cells;
    iii. fragments of inactivated *P.luminescens cells*; and
    iv. supernatant of cultures of *P.luminescens* cells or a fraction thereof possessing the insecticidal activity.

2. The insecticidal composition of claim 1, comprising living insecticidal bacteria.

3. The insecticidal composition of claim 1, comprising inactivated bacterial cells, fragments thereof or supernatant of the insecticidal bacterial culture.

4. The composition of any one of claims 1 to 3, wherein the *P.luminescens* bacteria have the characteristics of the strain XP01, CNCM I-1760.

5. A method for the treatment of insect infestation of plants, comprising administering to plants or to their environment a composition of any one of claims 1 to 4.

6. The method of claim 5, wherein said active ingredients are administered to aerial plant parts.

7. The method of claim 5, wherein said active ingredients are administered to soil.

8. A pure culture of bacteria, belonging to the species *P.luminescens* and having an insecticidal activity manifested by their ability to infect insects and exert insecticidal activity, without requiring a nematode as an infection vector.

9. The pure culture of claim 8, wherein said bacteria have the characteristics of the strain XP01, CNCM I-1760.

10. Bacteria of the strain XP01, CNCM I-1760.

Fig. 1

## EUROPEAN SEARCH REPORT

European Patent Office

Application Number

EP 96 11 2680

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | US-A-4 672 130 (S.H. RHODES ET AL.) 9 June 1987<br>* column 1, line 34 - line 40 *<br>* column 2, line 36 - line 50 *<br>--- | 1,3,5-7 | A01N63/00<br>C12N1/20<br>//(C12N1/20,<br>  C12R1:01) |
| A | WO-A-85 03412 (BIOTECHNOLOGY AUSTRALIA) 15 August 1985<br>* page 1, line 14 - line 25 *<br>----- | 1-7 | |
| | | | TECHNICAL FIELDS SEARCHED (Int.Cl.6)<br><br>A01N<br>C12N |

The present search report has been drawn up for all claims

| Place of search | Date of completion of the search | Examiner |
|---|---|---|
| THE HAGUE | 8 January 1997 | Decorte, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)